(19) Europäisches Patentamt
European Patent Office
Office européen des brevets

(11) Veröffentlichungsnummer: **0 124 482**
**B1**

(12) # EUROPÄISCHE PATENTSCHRIFT

(45) Veröffentlichungstag der Patentschrift: **08.11.89**

(51) Int. Cl.⁴: **C 07 D 233/60, C 08 G 59/18**

(21) Anmeldenummer: **84810192.9**

(22) Anmeldetag: **19.04.84**

(54) **Neue Imidazolide und deren Verwendung als Härter für Polyepoxidverbindungen.**

(30) Priorität: **29.04.83 CH 2316/83**

(43) Veröffentlichungstag der Anmeldung: **07.11.84 Patentblatt 84/45**

(45) Bekanntmachung des Hinweises auf die Patenterteilung: **08.11.89 Patentblatt 89/45**

(84) Benannte Vertragsstaaten: **CH DE FR GB IT LI NL SE**

(56) Entgegenhaltungen:
**JP-B-49 007 599**
**US-A- 4 343 923**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Schmid, Rolf, Dr., Buhnenstock 14, CH-3150 Schwarzenburg (CH)**
Erfinder: **Zondler, Helmut, Dr., Oberwilerstrasse 49, CH-4103 Bottmingen (CH)**
Erfinder: **Fischer, Michael, Dr., Allmendstrasse, CH-1712 Tafers (CH)**
Erfinder: **Stauffer, Werner, Avenue Jean-Marie Musy 6, CH-1700 Fribourg (CH)**

**EP 0 124 482 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Imidazolide (N-Acylimidazole), ein Verfahren zu ihrer Herstellung sowie deren Verwendung als Härter für Polyepoxidverbindungen mit durchschnittlich mehr als einer Epoxidgruppe im Molekül.

Es ist bekannt, bei der Härtung von Epoxidharzen Imidazole als Härter einzusetzen. So beschreibt z.B. die JP Auslegeschrift 49-7599 N-Acylimidazole, die am Imidazolring Halogenatome, Äther-, Thioäther-, Cyclohexyl- oder unsubstituierte oder mit einer Ester- oder Amidgruppe substituierte Alkylgruppen tragen können, wie z.B. 1-(2-Chlorbenzoyl)-imidazol und Trimethylbenzoylimidazol, als Härter. Gemische von Polyepoxiden mit solchen Imidazoliden können bei Raumtemperatur unter Ausschluß von Wasser einige Zeit gelagert und bei erhöhter Temperatur durch Aufnahme von Wasserdampf aus der Luft gehärtet werden.

Diese vorbekannten Systeme lassen bezüglich der Durchhärtung bei offener Oberfläche, wie sie beispielsweise bei der Herstellung von Beschichtungen, umwickelten Rohren oder Prepregs erforderlich ist, und/oder der Stabilität noch zu wünschen übrig.

Es wurden nun neue Imidazolide gefunden, die sich u.a. durch sehr gute Durchhärtung bei offener Oberfläche, verbunden mit verbesserten Stabilitätseigenschaften und längeren Verarbeitungszeiten auszeichnen.

Gegenstand vorliegender Erfindung sind daher Imidazolide der Formel I

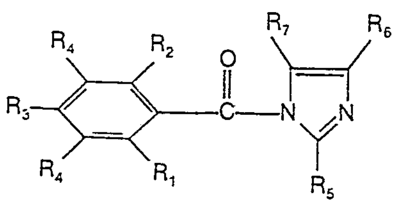

(I),

worin

$R_1$ Methyl, Äthyl oder Isopropyl bedeutet,

$R_2$, $R_3$ und $R_4$ unabhängig voneinander für Wasserstoff, Methyl, Äthyl oder Isopropyl stehen, unter der Bedingung, daß $R_2$ nur dann Wasserstoff bedeutet, wenn $R_1$ Isopropyl ist,

$R_5$, $R_6$ und $R_7$ unabhängig voneinander Wasserstoff, Methyl, Äthyl, Isopropyl, unsubstituiertes oder mit Methyl- oder Äthylgruppen substituiertes Phenyl bedeuten, unter der Bedingung, daß mindestens eines der Symbole $R_5$, $R_6$ und $R_7$ für ein unsubstituiertes oder definitionsgemäß substituiertes Phenyl steht.

Bedeuten $R_5$, $R_6$ und $R_7$ ein mit Methyl- oder Äthylgruppen substituiertes Phenyl, so kommen z.B. 2-Methylphenyl, 2-Äthylphenyl, 2,6-Diäthylphenyl und 2,4,6-Trimethylphenyl in Frage.

Bevorzugt sind Imidazolide der Formel I, worin $R_1$, $R_2$ und $R_3$ Methyl und $R_4$ Wasserstoff bedeuten.

Ebenfalls bevorzugt sind Imidazolide der Formel I, worin eines der Symbole $R_5$, $R_6$ und $R_7$ ein unsubstituiertes Phenyl und die beiden anderen Wasserstoff bedeuten. Besonders bevorzugt sind diejenigen, worin $R_5$ Phenyl, $R_6$ und $R_7$ Wasserstoff bedeuten.

Besonders bevorzugt ist die Verbindung 1-(2,4,6-Trimethylbenzoyl)-2-phenylimidazol.

Die erfindungsgemäßen Imidazolide der Formel I kann man z.B. dadurch erhalten, daß man ein Säurehalogenid der Formel II

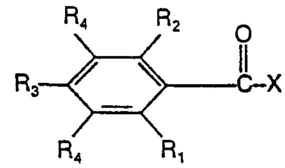

(II)

mit einem Imidazol der Formel III

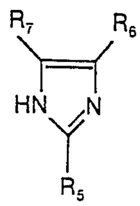

(III),

worin X Chlor oder Brom entspricht, und die Symbole $R_1$, $R_2$, $R_3$, $R_4$, $R_5$, $R_6$ und $R_7$ die zuvor genannte Bedeutung haben, in Gegenwart eines Säureakzeptors umsetzt.

Als Säureakzeptoren können die üblicherweise verwendeten Substanzen, wie z.B. tertiäre Amine, insbesondere Triäthylamin, Pyridinbasen, oder das Imidazol der Formel III in molarem Überschuß eingesetzt werden.

Die Umsetzung erfolgt vorteilhafterweise in einem inerten organischen Lösungsmittel. Als Lösungsmittel eignen sich z.B. aromatische Kohlenwasserstoffe, wie z.B. Toluol oder Xylol; halogenierte aliphatische oder aromatische Kohlenwasserstoffe, wie z.B. $CCl_4$, $HCCl_3$, $CH_2Cl_2$; Äthylenchlorid oder Chlorbenzol, Dichlorbenzol oder Chlornaphthalin; Äther, wie z.B. Diäthyläther, Diisopropyläther, Dioxan oder Tetrahydrofuran.

Die Reaktion wird zweckmäßig im Temperaturbereich von 0 °C bis 150 °C durchgeführt.

Die als Ausgangsverbindungen verwendeten Säurehalogenide und Imidazole sind im Handel erhältlich oder können nach bekannten Methoden hergestellt werden.

Die erfindungsgemäßen Imidazolide eignen sich hervorragend als Härter für Epoxidharze. Ein weiterer Gegenstand vorliegender Erfindung sind daher härtbare Gemische, welche mindestens ein Imidazolid der Formel I und eine Polyepoxidverbindung mit durchschnittlich mehr als einer Epoxidgruppe im Molekül enthalten.

Das Mischungsverhältnis kann so gewählt werden, daß die härtbaren Gemische 2 bis 15 Gewichtsteile Imidazolid der Formel I auf 100 Gewichtsteile Polyepoxidverbindung enthalten.

Als Polyepoxidverbindungen kommen für die erfindungsgemäßen härtbaren Gemische solche mit durchschnittlich mehr als einer an ein Heteroatom, vorzugsweise Sauerstoff oder Stickstoff, gebundenen Glycidylgruppe oder β-Methylglycidylgruppe oder solche mit durchschnittlich mehr als einer Epoxycyclohexylgruppierung in Frage. Genannt seien beispielsweise:

a) Di- bzw. Polyglycidyläther von mehrwertigen aliphatischen Alkoholen, wie 1,4-Butandiol, Neopentylglykol, oder von Polyalkylenglykolen, wie Polypropylenglykolen,

b) Di- oder Polyglycidyläther von cycloaliphatischen Polyolen, wie 2,2-Bis-(4-hydroxycyclohexyl)-propan, 1,4-Bis-(hydroxymethyl)-cyclohexan,

c) Verbindungen mit Epoxycyclohexylgruppierungen, wie 3,4-Epoxycyclohexylcarbonsäure-3′,4′-epoxycyclohexylmethyl-ester, 3-(3′,4′-Epoxycyclohexyl)-2,4-dioxa-spiro-[5,5]-8,9-epoxyundecan, Adipinsäure-bis-(3,4-epoxycyclohexylmethyl)-ester,

d) Di- bzw. Polyglycidylester mehrwertiger Carbonsäuren, wie Phthalsäure, Terephthalsäure, $\Delta^4$-Tetrahydrophthalsäure, Hexahydrophthalsäure, Trimellitsäure, Oxalsäure, Malonsäure, Adipinsäure, Bernsteinsäure, Fumarsäure, Maleinsäure,

und bevorzugt:

e) Di- bzw. Polyglycidyläther von mehrwertigen Phenolen, wie Resorcin, Bis-(p-hydroxyphenyl)-methan, 2,2-Bis-(p-hydroxyphenyl)-propan (= Bisphenol A), 2,2-Bis-(4′-hydroxy-3′,5′-dibromphenyl)propan, 1,1,2,2-Tetrakis-(p-hydroxyphenyl)äthan, oder von unter sauren Bedingungen erhaltenen Kondensationsprodukten von Phenolen mit Formaldehyd, wie Phenol-Novolaken oder Kresol-Novolaken, insbesondere die mittel- bis niederviskosen Novolake,

f) N-Glycidylderivate von Aminen, Amiden und heterocyclischen Stickstoffbasen, wie N,N-Diglycidylanilin, N,N-Diglycidyltoluidin, N,N,N′,N′-Tetraglycidyl-bis-(p-aminophenyl)methan; Triglycidylverbindung des p-Hydroxyanilins; Triglycidylisocyanurat; N,N′-Diglycidyläthylenharnstoff; N,N′-Diglycidyl-5,5-dimethylhydantoin, N,N′-Diglycidyl-5-isopropylhydantoin; N,N′-Diglycidyl-5,5-dimethyl-6-isopropyl-5,6-dihydro-uracil.

Es können auch Mischungen der genannten Di- und Polyepoxide eingesetzt werden.

Besonders bevorzugt enthält das härtbare Gemisch als Polyepoxidverbindung einen Bisphenol-A-diglycidyläther, einen Polygylcidyläther von Phenol- oder Kresol-Novolaken oder eine N-Glycidylverbindung, insbesondere N,N,N′,N′-Tetraglycidyl-bis-(p-aminophenyl)methan, und als Imidazolid 1-(2,4,6-Trimethylbenzoyl)-2-phenylimidazol.

Die Härtung der erfindungsgemäßen härtbaren Mischungen zu Formkörpern und dergleichen erfolgt zweckmäßig im Temperaturintervall von 50°–250 °C, vorzugsweise zwischen 100° und 200 °C. Man kann die Härtung in bekannter Weise auch zwei- oder mehrstufig durchführen, wobei die erste Härtungsstufe bei niedriger Temperatur und die Nachhärtung bei höherer Temperatur durchgeführt werden, und zwar vorzugsweise bei Temperaturen, die der Glasumwandlungstemperatur des vollständig ausgehärteten Harzes entsprechen.

Wenn die Gelier- bzw. Härtungszeiten verkürzt werden sollen, können bekannte Härtungskatalysatoren zugesetzt werden. Geeignete Katalysatoren sind z.B. aliphatische Alkohole und Phenole, vorzugsweise mehrwertige phenolische Verbindungen, wie Bisphenol A und Pyrogallol. Besonders vorteilhaft ist die Verwendung von Polyepoxiden, wie aufgezählt unter a) und e), welche noch freie Hydroxylgruppen enthalten.

Die Katalysatoren können in Mengen von 1 bis 12 Gew.-%, vorzugsweise 3 bis 8 Gew.-%, bezogen auf das Reaktionsgemisch, eingesetzt werden.

Die erfindungsgemäßen härtbaren Mischungen können ferner vor der Härtung in irgendeiner Phase mit üblichen Modifizierungsmitteln, wie Streck-, Füll- und Verstärkungsmitteln, Pigmenten, Farbstoffen, organischen Lösungsmitteln, Weichmachern, Verlaufmitteln, Thixotropiermitteln, Flexibilisatoren, flammhemmenden Stoffen oder Formtrennmitteln, versetzt werden.

Als Streckmittel, Verstärkungsmittel, Füllmittel und Pigmente, die in den erfindungsgemäßen härtbaren Mischungen eingesetzt werden können, seien z.B. genannt: Steinkohlenteer, Bitumen, flüssige Cumaron-Inden-Harze, Textilfasern, Glasfasern, Asbestfasern, Borfasern, Kohlenstofffasern, Cellulose, Polyäthylenpulver, Polypropylenpulver, Quarzmehl, mineralische Silikate, wie Glimmer, Asbestmehl, Schiefermehl, Kaolin, Kieselsäureaerogel, Lithopone, Schwerspat, Titandioxid, Ruß, Graphit, Oxidfarben, wie Eisenoxid, oder Metallpulver, wie Aluminiumpulver oder Eisenpulver.

Als organische Lösungsmittel eignen sich für die Modifikation der härtbaren Mischungen z.B. Toluol, Xylol, Butylacetat, Aceton und Methyläthylketon.

Als Weichmacher können für die Modifizierung der härtbaren Mischungen z.B. Dibutyl-, Dioctyl- und Dinonylphthalat, Trikresylphosphat, Trixylenylphosphat und Diphenoxyäthylformal eingesetzt werden.

Als Verlaufmittel beim Einsatz der härtbaren Mischungen speziell im Oberflächenschutz, kann man z.B. Silikone, flüssige Acrylharze, Celluloseacetobutyrat, Polyvinylbutyral, Wachse, Stearate, etc. (welche z.T. auch als Formtrennmittel Anwendung finden) zusetzen.

Als Flexibilisatoren eignen sich z.B. Oligoestersegmente, Polyester, Thermoplaste und Butadien-Acrylnitril-Oligomere, wie Hycar® der Firma Ciago.

Die Herstellung der erfindungsgemäßen härtbaren Gemische kann in üblicher Weise mit Hilfe bekannter Mischaggregate (Rührer, Kneter, Walzen oder im Falle fester Substanzen bzw. Pulver in Mühlen oder Trockenmischern) erfolgen. In manchen Fällen ist ein kurzes Erwärmen des Gemisches erforderlich, um eine genügene Homogenität zu erreichen.

Die erfindungsgemäßen härtbaren Gemische zeichnen sich durch gute Lagerstabilität, lange Verarbeitungszeiten, hohe Wärmeformbeständigkeit, gute Heißwasser- und Chemikalien-Beständigkeit, gute Wärmebeständigkeit und durch gute Durchhärtung auch bei offener Härtung in dünner Schicht aus.

Die erfindungsgemäßen härtbaren Gemische finden ihren Einsatz vor allem auf den Gebieten des Oberflächenschutzes, der Elektrotechnik, der Laminierverfahren, der Klebstofftechnik und im Bauwesen. Sie können in jeweils dem speziellen Anwendungszweck angepaßter Formulierung, im ungefüllten oder gefüllten Zustand, gegebenenfalls in Form von Lösungen oder Emulsionen, als Anstrichmittel, lösungsmittelfreie Beschichtungen, Sinterpulver, Preßmassen, Spritzgußformulierungen, Tauchharze, Gießharze, Schaumstoffe, Filme, Folien, Bindemittel, Werkzeugharze, Laminierharze, Dichtungs- und Spachtelmassen, Bodenbelagsmassen und Bindemittel für mineralische Aggregate verwendet werden.

Besonders vorteilhaft werden die erfindungsgemäßen härtbaren Gemische als Wickel- oder Imprägnierharze für faserverstärkte Verbundstoffe, wie zur Herstellung von heißwasserbeständigen Rohren und Behältern, als Matrix-Komponente für hochwertige Verbundstoffe und zum Verkleben von Kunststoffen, Verbundstoffen und Metallen eingesetzt.

Beispiel A
Herstellung von
1-(2,4,6-Trimethylbenzoyl)-2-phenylimidazol

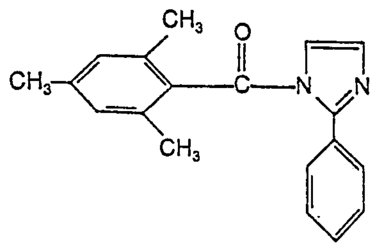

144,2 g (1,00 Mol) 2-Phenylimidazol werden bei 90 °C in 900 ml Toluol gelöst. Zu dieser Lösung fügt man 104,2 g (1,03 Mol) Triäthylamin zu und läßt bei 90 °C eine Lösung von 182,6 g (1,00 Mol) 2,4,6-Trimethylbenzoylchlorid in 300 ml Toluol innerhalb von 2 Stunden zutropfen, wobei sich Triäthylaminhydrochlorid abscheidet. Dieses wird bei Zimmertemperatur abgesaugt und mit Toluol ausgewaschen. Nach dem Einengen des Filtrats erhält man 296 g Rohprodukt. Dessen Umkristallisation aus 580 ml Acetonitril ergibt 121,2 g einer 1. Fraktion (Smp. 94,5–96 °C), 82,1 g einer 2. Fraktion (Smp. 94,5–96 °C) und 24,0 g einer 3. Fraktion (Smp. 94–95 °C), insgesamt 227,3 g an 1-(2,4,6-Trimethylbenzoyl)-2-phenylimidazol (78,3% d. Theorie).

Anwendungsbeispiele B
Die folgenden Härter und Polyepoxidverbindungen werden eingesetzt:

Härter:

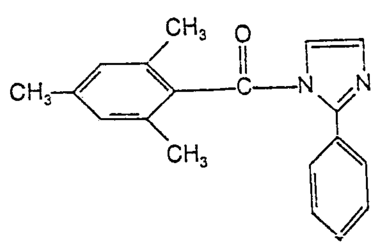

Epoxidharz I: mittel- bis niedrigviskoses Epoxid-Phenol-Novolakharz mit einem Epoxid-Äquivalentgewicht von 175,5 g/Äq.

Epoxidharz II: N,N,N',N'-Tetraglycidyl-bis-(p-aminophenyl)methan.

Beispiel 1

20 g Epoxidharz I werden auf 100 °C erwärmt, mit 1,2 g Härter gut vermischt und entgast. 2 g der noch warmen Mischung werden in eine offene Aluminium-Schale von 60 mm Durchmesser, der Rest in eine Aluminium-Form mit den Abmessungen 80 × 10 × 2 mm gegossen. Es wird während 2 Stunden bei 120 °C und anschließend während 3 Stunden bei 180 °C gehärtet, und die Glasübergangstemperatur Tg' wird nach der thermomechanischen Analyse mit belastetem Stempel als Maximum der Eindringgeschwindigkeit gemessen.

Tg' der Al-Schale: 145 °C
Tg' der Al-Form: 165 °C.

Die hohe Glasumwandlungstemperatur ist ein

Maß für die gute Durchhärtung bei offener Oberfläche.

Beispiel 2

100 g Epoxidharz I werden in der Wärme mit 6 g Härter gemischt, entgast und in Aluminium-Formen der Dimensionen 200 × 200 × 4 mm gegossen. Die Härtung erfolgt während je 2 Stunden bei 140 °C und anschließend bei 180 °C. Aus den erhaltenen Platten werden Prüfkörper mit den Abmessungen 80 × 10 × 4 mm geschnitten und die Biegefestigkeit (ISO 178), die Randdehnung im Anlieferungszustand und nach Alterung an der Luft und in Wasser sowie die Wasseraufnahme bestimmt. Die Ergebnisse sind in Tabelle I zusammengefaßt.

Tabelle I

| Vorbehandlung | Biegefestigkeit MPa | Randdehnung % | Wasseraufnahme Gew.-% |
|---|---|---|---|
| Bei der Anlieferung | 92 | 4,0 | – |
| nach 10 Tagen | 97 | 4,4 | – |
| an der Luft bei 160 °C | | | |
| nach 30 Tagen | 103 | 4,6 | – |
| nach 10 Tagen | 63 | 2,5 | 2,0 |
| in Wasser bei 120 °C | | | |
| nach 30 Tagen | 69 | 2,2 | 2,2 |

Das Harz-Härter-Gemisch zeigt ferner bei 75 °C eine dynamische Viskosität von 210 mPa · s. Nach 8 Stunden bei 75 °C steigt die Viskosität nur bis 350 mPa · s an. Das System ist somit besonders geeignet zur Verwendung als Imprägnierharz, und es härtet bei mäßig hohen Temperaturen (größtenteils schon bei 140 °C) durch.

Beispiel 3

Beispiel 2 wird wiederholt, jedoch mit dem Unterschied, daß man 80 g Epoxidharz I zusammen mit 20 g Epoxidharz II verwendet. Nach der Härtung werden die folgenden Eigenschaften gemessen:

Tg': 156°C bei der Härtung eines Films an der Luft; 181 °C bei der Härtung in der geschlossenen Form.

Dynamische Viskosität bei 75 °C: 310 mPa · s.

Nach der Härtung werden die folgenden Eigenschaften im Anlieferungszustand gemessen:
Biegefestigkeit: 108 MPa
Randdehnung: 5,9%.

**Patentansprüche**

1. Imidazolide der Formel I

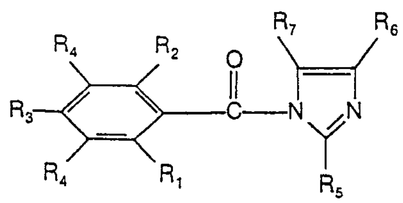

(I),

worin

$R_1$ Methyl, Äthyl oder Isopropyl bedeutet,

$R_2$, $R_3$ und $R_4$ unabhängig voneinander für Wasserstoff, Methyl, Äthyl oder Isopropyl stehen, unter der Bedingung, daß $R_2$ nur dann Wasserstoff bedeutet, wenn $R_1$ Isopropyl ist,

$R_5$, $R_6$ und $R_7$ unabhängig voneinander Wasserstoff, Methyl, Äthyl, Isopropyl, unsubstituiertes oder mit Methyl- oder Äthylgruppen substituiertes Phenyl bedeuten, unter der Bedingung, daß mindestens eines der Symbole $R_5$, $R_6$ und $R_7$ für ein unsubstituiertes oder definitionsgemäß substituiertes Phenyl steht.

2. Imidazolide gemäß Anspruch 1, worin $R_1$, $R_2$ und $R_3$ Methyl und $R_4$ Wasserstoff bedeuten.

3. Imidazolide gemäß Anspruch 1, worin eines der Symbole $R_5$, $R_6$ und $R_7$ ein unsubstituiertes Phenyl und die beiden anderen Wasserstoff bedeuten.

4. Imidazolide gemäß Anspruch 1, worin $R_5$ Phenyl, $R_6$ und $R_7$ Wasserstoff bedeuten.

5. Ein Imidazolid gemäß Anspruch 1, dadurch gekennzeichnet, daß es 1-(2,4,6-Trimethylbenzoyl)-2-phenylimidazol ist.

6. Verfahren zur Herstellung von Imidazoliden der Formel I gemäß Anspruch 1, dadurch gekennzeichnet, daß man ein Säurehalogenid der Formel II

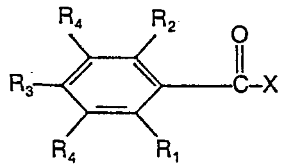

(II)

mit einem Imidazol der Formel III

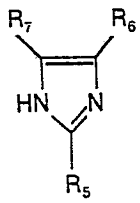

(III),

worin X Chlor oder Brom entspricht, und die Symbole $R_1$, $R_2$, $R_3$, $R_4$, $R_5$, $R_6$ und $R_7$ die in Anspruch 1 genannte Bedeutung haben, in Gegenwart eines Säureakzeptors umsetzt.

7. Härtbare Gemische, welche mindestens ein Imidazolid der Formel I gemäß Anspruch 1 und eine Polyepoxidverbindung mit durchschnittlich mehr als einer Epoxidgruppe im Molekül enthalten.

8. Härtbare Gemische gemäß Anspruch 7, welche als Polyepoxidverbindung einen Bisphenol-A-diglycidyläther, einen Polyglycidyläther von Phenol- oder Kresol-Novolaken oder eine N-Glycidylverbindung enthalten.

9. Härtbare Gemische gemäß Anspruch 7, worin das Imidazolid 1-(2,4,6-Trimethylbenzoyl)-2-phenylimidazol ist.

10. Die unter Verwendung eines Gemisches nach Anspruch 7 durch Härten erhaltenen Produkte.

**Claims**

1. An imidazolide of the formula I

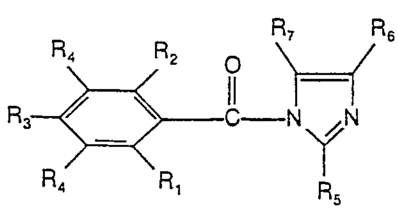

(I),

in which

$R_1$ is methyl, ethyl or isopropyl,

$R_2$, $R_3$ and $R_4$ independently of one another are each hydrogen, methyl, ethyl or isopropyl, with the proviso that $R_2$ is hydrogen only when $R_1$ is isopropyl,

$R_5$, $R_6$ and $R_7$ independently of one another are each hydrogen, methyl, ethyl, isopropyl or phenyl which is unsubstituted or substituted by methyl or ethyl groups, with the proviso that at least one of the symbols $R_5$, $R_6$ and $R_7$ is phenyl which is unsubstituted or substituted according to definition.

2. An imidazolide according to claim 1, in which $R_1$, $R_2$ and $R_3$ are methyl, and $R_4$ is hydrogen.

3. An imidazolide according to claim 1, in which one of the symbols $R_5$, $R_6$ and $R_7$ is an unsubstituted phenyl, and the two others are hydrogen.

4. An imidazolide according to claim 1, in which $R_5$ is phenyl, and $R_6$ and $R_7$ are hydrogen.

5. An imidazolide according to claim 1, which is 1-(2,4,6-trimethylbenzoyl)-2-phenylimidazole.

6. A process for producing an imidazolide of the formula I according to claim 1, which comprises reacting an acid halide of the formula II

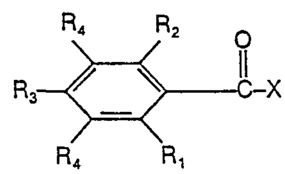

(II)

in the presence of an acid acceptor, with an imidazole of the formula III

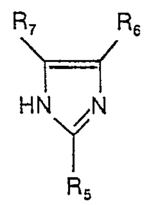

(III),

in which X is chlorine or bromine, and the symbols $R_1$, $R_2$, $R_3$, $R_4$, $R_5$, $R_6$ and $R_7$ have the meanings given in claim 1.

7. A curable mixture which contains at least one imidazolide of the formula I according to claim 1 and a polyepoxide compound having on average more than one epoxy group in the molecule.

8. A curable mixture according to claim 7, which contains, as the polyepoxide compound, a bisphenol-A-diglycidyl ether, a polyglycidyl ether of phenol or cresol novolaks or an N-glycidyl compound.

9. A curable mixture according to claim 7, in which the imidazolide is 1-(2,4,6-trimethylbenzoyl)-2-phenylimidazole.

10. A product obtained by curing using a mixture according to claim 7.

**Revendications**

1. Imidazolides répondant à la formule I:

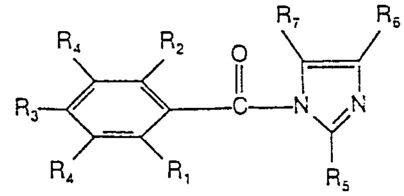

(I),

dans laquelle:

$R_1$ représente un radical méthyle, éthyle ou isopropyle,

$R_2$, $R_3$ et $R_4$ représentent chacun, indépendamment les uns des autres, l'hydrogène ou un radical méthyle, éthyle ou isopropyle, avec la condition que $R_2$ ne soit l'hydrogène que dans le cas où $R_1$ est un radical isopropyle, et

$R_5$, $R_6$ et $R_7$ représentent chacun, indépendamment les uns des autres, l'hydrogène, un radical méthyle, éthyle ou isopropyle, ou un radical phényle non substitué ou porteur de radicaux méthyles ou éthyles, avec la condition qu'au moins un des symboles $R_5$, $R_6$ et $R_7$ représente un phényle non substitué ou substitué conformément à la définition.

2. Imidazolides selon la revendication 1 dans lesquels $R_1$, $R_2$ et $R_3$ représentent chacun un méthyle et $R_4$ représente l'hydrogène.

3. Imidazolides selon la revendication 1 dans lesquels un des symboles $R_5$, $R_6$ et $R_7$ représente un phényle non substitué et les deux autres représentent chacun l'hydrogène.

4. Imidazolides selon la revendication 1 dans lesquels $R_5$ représente un phényle, et $R_6$ et $R_7$ représentent chacun l'hydrogène.

5. Imidazolide selon la revendication 1, en l'espèce le (triméthyl-2,4,6 benzoyl)-1 phényl-2 imidazole.

6. Procédé pour préparer des imidazolides de formule I selon la revendication 1, procédé caractérisé en ce qu'on fait réagir un halogénure d'acide de formule II:

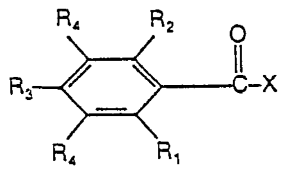

(II)

avec un imidazole de formule III:

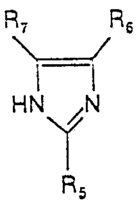

(III),

formules dans lesquelles X représente le chlore ou le brome, et les symboles $R_1$, $R_2$, $R_3$, $R_4$, $R_5$, $R_6$ et $R_7$ ont les significations qui leur ont été attribuées à la revendication 1, en présence d'un accepteur d'acides.

7. Mélanges durcissables qui contiennent au moins un imidazolide de formule I selon la revendication 1 et un composé polyépoxydique renfermant en moyenne plus d'un radical époxy par molécule.

8. Mélanges durcissables selon la revendication 7 qui contiennent, comme composé polyépoxydique, un éther diglycidylique du bis-phénol A, un éther polyglycidylique d'une novolaque phénolique ou crésolique, ou un composé N-glycidylique.

9. Mélanges durcissables selon la revendication 7 dans lesquels l'imidazolide est le (triméthyl-2,4,6 benzoyl)-1 phényl-2 imidazole.

10. Produits que l'on a obtenus par durcissement en utilisant un mélange selon la revendication 7.